# EUROPEAN PATENT APPLICATION

(11) **EP 2 057 946 A1**
(43) Date of publication of application: **13.05.2009**
(21) Application number: 07806114.0
(22) Date of filing: 28.08.2007
(51) Int. Cl.: A61B 17/06

(54) **EYELESS SUTURE NEEDLE AND METHOD OF MANUFACTURING THE SAME**

(30) Priority: 31.08.2006 JP 2006234726
(71) Applicant: MANI Inc., Utsunomiya-shi, Tochigi 321-3231 (JP)
(72) Inventor: AKUTSU, Shinichi, Tochigi 321-3231 (JP); MATSUTANI, Kanji, Tochigi 321-3231 (JP); MASHIKO, Masaki, Tochigi 321-3231 (JP)
(74) Representative: Peckmann, Ralf
(86) International application number: PCT/JP2007/066592
(87) International publication number: WO 2008/026558

(57) **Abstract**

A simple fabrication method for a suture needle coated with silicone, which prevents silicone from penetrating into a hole is provided.

The fabrication method for a suture needle coated with silicone includes the steps of: facing a hole 12 formed at a base of an eyeless suture needle 10 downward and supporting it with a gripping unit; and coating silicone on the entire outside of the eyeless suture needle, making the inner wall of the hole be the base material of the eyeless suture needle. Consequently, the silicone can be prevented from intruding into the hole by a simple method.

## Description

### Technical Field

The present invention relates to an eyeless suture needle. It particularly relates to a silicone coated eyeless suture needle and a fabrication method for the same.

### Background Art

Suture needles thrust a patient's living body tissue, and since pain afflicting the patient increases when thrust resistance is great, processing to reduce the thrust resistance is carried out. Silicone is generally applied as a method of reducing thrust resistance; more specifically, suture needles are put in a basket spread apart and soaked in a silicone solution.

Meanwhile, the eyeless suture needle is fixed by forming a hole in a base end of a suture needle along the length thereof, inserting the end of a suture thread therein, and crimping the hole from the outside. In the case of coating such eyeless suture needles with silicone, when they are put in a basket spread apart and soaked in a silicone solution, the silicone solution penetrates into the holes, coating the inner wall of the holes with the silicone. Coating of the inner wall of the holes with silicone makes it slippery between the inner wall and the suture thread. Therefore, even if the suture thread is crimped, it slips off easily, which is a problem when it is used in surgery.

As a result, a method of putting water into the hole so that silicone does not get in (refer to Patent Document 1, for example), and a method of removing the silicone that got into the hole using a solvent (Patent Document 2, for example) have been proposed. Moreover, a method of coating silicone and then forming a hole has been proposed in Patent Document 3.
Patent Document 1: Japanese Published Unexamined Application No. Hei 4-317644
Patent Document 2: Patent No. 3124564
Patent Document 3: Patent No. 3471004

### Disclosure of invention

### [Problem to be solved by the invention]

However, the method of putting water into the hole so that silicone does not get in of Patent Document 1, and the method of removing the silicone that got into the hole using a solvent of Patent Document 2 presents cumbersome work with an increase in the number of steps.

The present invention is devised in consideration of such problems, and provides a simple fabrication method for a suture needle coated with silicone, which prevents silicone from penetrating into the hole.

### [Means of solving the problem]

In order to achieve the aforementioned objective, a fabrication method for an eyeless suture needle according to the present invention is **characterized in that** it includes the steps of: supporting an eyeless suture needle with a gripping unit; and facing a hole at a base of the supported eyeless suture needle downward and coating with silicone such that the silicone does not coat inside of the hole.

In addition, an eyeless suture needle according to the present invention is **characterized in that** it is coated with silicone, the coating of the outside portion of a hole of the eyeless suture needle and part of the main body is unsmooth, the coating of other parts is smooth, and the inner wall of the hole is not coated with silicone.

The gripping unit may be made of a metal and hold the eyeless suture needle with the help of the energization force of an elastic body, and support the outside of a hole of the eyeless suture needle while a holding fixture may hold near the middle of a main body of the eyeless suture needle.

### [Effects of invention]

According to the fabrication method for an eyeless suture needle coated with silicone according to the present invention, an eyeless suture needle smoothly coated with silicone on the outside and not coated inside the hole may be provided.

Moreover, since the eyeless suture needle according to the present invention has an unsmooth coating on the outside of the hole of the eyeless suture needle and part of the main body, and smooth coating on other parts, thrust resistance may be reduced, thereby reducing the burden on a patient. In other words, since the outside of the hole or the area to be crimped and part of the main body are supported and coated in order to surely coat the needlepoint and coat as much of the outer surface of the suture needle as possible, it is not coated smoothly.

Furthermore, since the suture thread is crimped with the inner wall of the hole not coated with silicone, the suture thread may be gripped tightly, and the suture thread slipping off while suturing live body tissue may be prevented.

### Brief Description of Drawings

FIG. 1 is an oblique perspective of an eyeless suture needle according to the present invention;
FIG. 2 is a magnified cross section of a base end of the eyeless suture needle of FIG. 1;
FIG. 3(a) is a diagram of the eyeless suture needle in a held state, and (b) is a cross section cut along line A-A of (a); and
FIG. 4 is a graph showing a relationship between angle α in FIG. 3 and thread snap-off strength of suture thread.

### [Description of reference numerals]

- 10:: Eyeless suture needle
- 11:: Main body
- 12:: Hole
- 20:: Suture thread
- 30:: Gripping unit
- a:: Central axis of hole
- b:: Perpendicular line in the direction of gravitational force
- α:: Angle

### Best mode of carrying out the invention

An embodiment according to the present invention is described with reference to attached drawings forthwith.

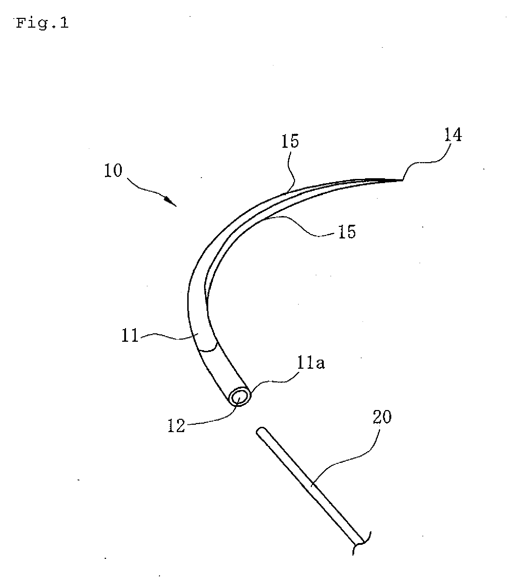

FIG. 1 is an oblique perspective of an eyeless suture needle according to the present invention. Since this eyeless suture needle 10 is fabricated using either a conventional or an already known method, the present invention is characterized by the silicone coating method.

As shown in FIG. 1, an eyeless suture needle 10 has a base end 11a on an end of a main body 11, and a hole 12 is formed in this base end 11a through a laser treatment, electron beam processing, electric discharge machining, a drill, or the like. Moreover, a sharp needlepoint 14 is formed at the tip of the eyeless suture needle 10, and a pyramid having a plurality of cutting blades 15 continuing from the needlepoint 14 is formed.

The eyeless suture needle 10 may be a sharpened suture needle in which the cross section of the cutting blades 15 as shown in FIG. 1 is formed into a polygon, a round needle (not shown in the drawing) without the cutting blades 15 having a cross section thereof formed in an approximate circle, or the like. These suture needles are selected and used according to live body tissue and region to be sutured.

Many types of suture thread 20 different in thickness and material (nylon, silk, etc.) and/or different in structure such as monofilament or multifilament are provided. An appropriate type of suture thread is selected and used according to live body tissue and region to be sutured. An end of the suture thread 20 is inserted in the hole 12, and the base end of the eyeless suture needle 10 is fixed by crushing and crimping the hole 12 using a press machine. The eyeless suture needle 10 has an advantage in that a thread long enough for suturing is fastened from the start, and thus there is no need to pass thread through a hole as with an eyed needle.

It is required during a suturing operation that the suture thread 20 attached to the eyeless suture needle 10 should not come out and snap off. In the case of an eyed needle, since suture thread is passed through a spring hole, two pieces of thread are lined up from both sides of the spring hole; however, the thread does not snap off as long as tension is not given to only one side.

However, with the eyeless suture needle, joining of the needle and thread is dependent on contact friction of the needle and the thread at the crimped portion, and loose crimping or a slippery inner wall of the hole could lead to disengagement of the thread from the suture needle while passing through living body tissue. On the other hand, crimping too tightly could lead to the thread partially breaking, decreasing in strength, and severing while passing through living body tissue. Therefore, the allowable thread snap-off strength of the eyeless suture needle 10 is preset according to the size of the suture thread 20 to be used, and a pull-off test is conducted to check whether the allowable thread snap-off strength is satisfied.

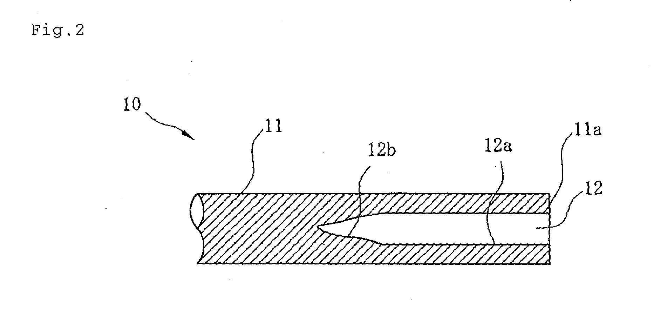

FIG. 2 is a magnified cross section of a base end of the eyeless suture needle 10 of FIG. 1. The base end 11a of the main body 11 has the hole 12 opened with a laser. The hole 12 has a part 12a somewhat wider than the diameter of the suture thread 20 and nearly straight so that the suture thread 20 can be inserted. A bottom part 12b gradually narrows, finally ending in a dead end. The suture thread 20 can be inserted only through the nearly straight part 12a. The diameter of the hole 12 is approximately 20 to 80% of diameter (diameter of the unprocessed main body 11) of the eyeless suture needle 10, and the depth of the nearly straight part 12a is approximately 1.1 to 10 times the diameter.

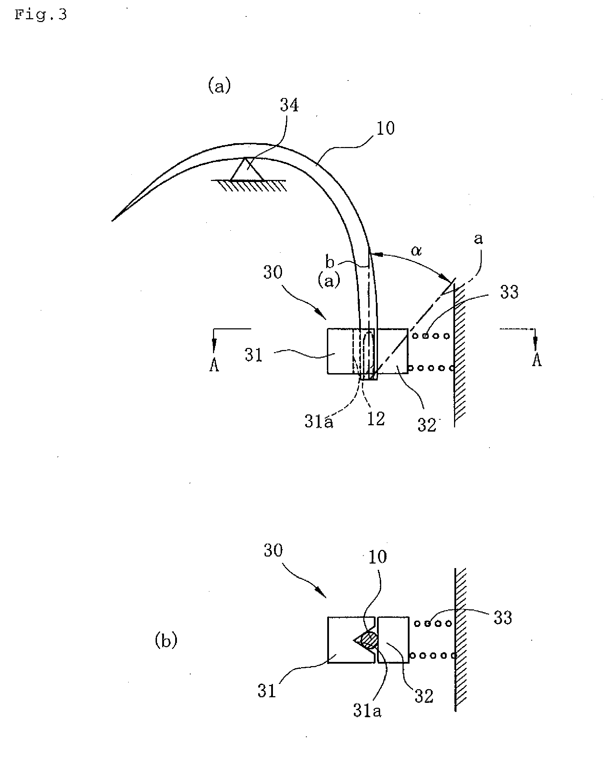

FIG. 3(a) is a diagram of the eyeless suture needle 10 in a held state, and (b) is a cross section cut along line A-A of (a). The eyeless suture needle 10 is held in open space by a gripping unit 30 with the hole 12 facing downward. The gripping unit 30 is made up of a V block 31 including a V groove 31a, a needle holding member 32, and an elastic body 33. The gripping unit 30 is preferably made of a material such as a metal or the like that is not affected by the silicone solution. The needle holding member 32 presses down on the eyeless suture needle 10 within the V groove 31a by the elastic body 33 such as a spring, holding the eyeless suture needle 10 by the frictional force thereof. In this embodiment, a supporting member 34 supporting at the middle of the main body 11 is also used. The reason for using this supporting member 34 is that it allows the gripping unit 30 to reduce the force of gripping the outside of the hole 12. In other words, when only supporting with the gripping unit 30, a certain amount of strength is necessary. However, it cannot grip strongly since the wall of the hole 12 is thin and the hole is subjected to annealing in order to improve efficiency of the crimping operation. Use of the supporting member 34 here allows support by a light force. Note that the fabrication method for the eyeless suture needle according to the present invention is not limited to this method, and controlling the posture of a robot to carry out the coating by soaking, spraying, and the like may be considered. A gripping unit for supporting the main body is not required in this case.

In this embodiment according to the present invention, the eyeless suture needle 10 has its base side upright, and central axis a of the hole 12 overlaps with perpendicular line b falling straight down. However, when the eyeless suture needle 10 is put at an angle, angle α made by the central axis a of the hole 12 and the perpendicular line b in the direction of gravitational force should be within the range of 0 to 45 degrees.

Once the eyeless suture needle 10 is held in this manner, the entire eyeless suture needle 10 is then soaked in the silicone solution in this state. Through this process, the entire outer surface of the eyeless suture needle 10 is coated with silicone.

Although the hole 12 is facing downward, since the surface tension of the hole 12 inlet increases relatively due to the air pressure within the hole 12 and the small diameter thereof, silicone does not get into the hole 12 or a very tiny bit of silicone enters the hole 12 inlet, and thus most of the inner wall of the hole 12 remains as the base material of the eyeless suture needle 10.

While the coated eyeless suture needle 10 is coated smoothly with silicone over the entire outside, portions making contact with the gripping unit 30 have areas to which silicone is not adhered and are rough surfaces. However, these portions make up a small portion of the entire eyeless suture needle 10, and do not increase the thrust resistance. Moreover, since the outside of the hole is crimped, the coating may become unsmooth in later steps anyway.

The simplest and shortest method of coating silicone is to soak in the silicone solution as in the above embodiment. Alternatively, a method of coating with a brush or by spraying with a spray gun may be used. However, with either method, silicone must be coated to sufficiently reach even the contact section between the gripping unit 30 and the eyeless suture needle 10.

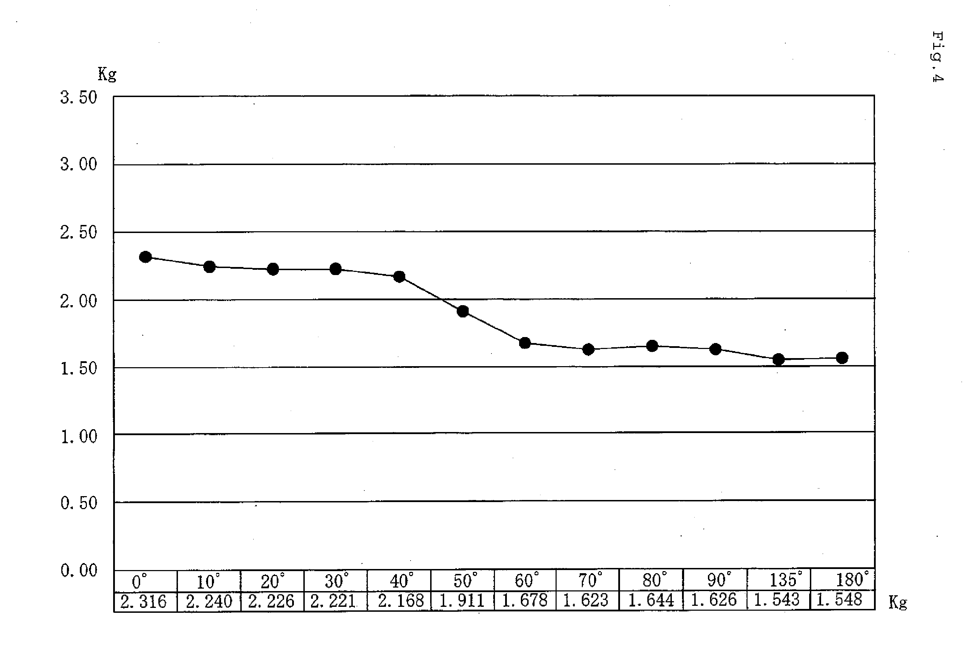

FIG. 4 is a graph showing a relationship between angle α in FIG. 3 and thread snap-off strength of the suture thread 20. Round needles with outer diameter of 0.78 mm and hole diameter of 0.47 mm and length of 45 mm are formed as eyeless suture needles 10 by changing the angle α shown in FIG. 3 and coating with silicone by soaking, size 0 (0.35 to 0.399 mm) suture thread used as the suture thread 20 is crimped and joined, and average values of thread snap-off strengths measured for thirty needles at each angle when a thread pull-off test is conducted thereafter are plotted.

While it is understood from FIG. 4 that the almost the same thread snap-off strength is maintained until the angle α reaches 40 degrees, once it hits 60 degrees, the thread snap-off strength decreases. Moreover, since the allowable thread snap-off strength for suture thread of this size is 1.5 Kg according to the USP standard, a thread snap-off strength of 2 Kg or greater is determined as sufficient, and thus angles up to 45 degrees are determined as providing sufficient thread snap-off strength.

## Claims

1. An eyeless suture needle, which is coated with silicone, wherein the coating of the outside of a hole of the eyeless suture needle and part of the main body of the eyeless suture needle is unsmooth, and the coating of other parts is smooth, and the inner wall of the hole is not coated with silicone.

2. An eyeless suture needle fabrication method, comprising the steps of: supporting an eyeless suture needle with a gripping unit; and facing a hole at a base of the supported eyeless suture needle downward and coating with silicone such that the silicone does not coat inside of the hole.

3. The eyeless suture needle fabrication method of Claim 2, wherein the gripping unit is made of a metal that is not affected by a silicone solution.

4. The eyeless suture needle fabrication method of either Claim 2 or 3, wherein the gripping unit is structured to grip by the energization force of an elastic body.

5. The eyeless suture needle fabrication method of any one of Claims 2 to 4, wherein the position supported by the gripping unit is set to outside of a hole of the eyeless suture needle.

6. The eyeless suture needle fabrication method of any one of Claims 2 to 5, further comprising the step of holding near the middle of the main body of the eyeless suture needle using a holding fixture.

7. The eyeless suture needle fabrication method of any one of Claims 2 to 6, wherein an angle formed by a central axis of the hole of the eyeless suture needle and a perpendicular line in the direction of gravitational force is within a range of 0 to 45 degrees.
